# EUROPEAN PATENT SPECIFICATION

(11) **EP 2 879 534 B1**
(45) Date of publication and mention of the grant of the patent: **29.03.2017**
(21) Application number: 13825422.2
(22) Date of filing: 02.08.2013
(51) Int. Cl.: A41B 17/00, A41B 9/04, A41B 9/00, A61F 13/84, A61F 13/66, A61F 13/496

(54) **MOISTURE-WICKING AND LEAK-RESISTANT UNDERWEAR GARMENTS**
FEUCHTIGKEITSAUFSAUGENDE UND LECKAGESICHERE UNTERWÄSCHE
SOUS-VÊTEMENTS ANTIFUITE ET ABSORBANT L'HUMIDITÉ

(30) Priority: 02.08.2012 US 201261678836 P
(43) Date of publication of application: 10.06.2015
(73) Proprietor: Thinx Inc., New York, NY 10001 (US)
(72) Inventor: DUNBAR Antonia, Saint, New York, NY 10001 (US); AGRAWAL, Miki, New York, NY 10001 (US); AGRAWAL, Radha, New York, NY 10001 (US)
(74) Representative: Casey, Alan Michael
(86) International application number: PCT/US2013/053518
(87) International publication number: WO 2014/022832

(56) References cited:
- EP-A1- 0 558 351
- WO-A1-94/10866
- US-A1- 2006 101 558
- US-A1- 2006 101 558
- US-A1- 2008 114 327
- US-A1- 2008 276 352
- US-A1- 2010 249 736
- US-A1- 2011 092 935
- US-A1- 2011 172 621

## Description

### CROSS-REFERENCE TO RELATED APPLICATIONS

### FIELD OF THE INTENTION

In various embodiments, the present invention relates generally to undergarments and, more specifically, to undergarments that absorb bodily fluids such as those associated with menstruation and incontinence.

### BACKGROUND

Women's undergarments may provide several functions, such as shaping the wearer's body, supporting parts of the body during exercise, and offering protection to outer clothing. Typically, a woman who is menstruating uses a tampon or a sanitary pad, in addition to wearing the undergarment, to keep outer garments from being soiled by bodily secretions and discharges. Although the tampon or pad may often absorb all of the liquid flow, unexpected leaks may still occur, in particular during the heavy-flow days of menstruation or during a change in body position.

An alternative solution for absorbing bodily secretions and discharges is an adult "brief," which offers a larger area of protection and may be particularly useful for women experiencing heavy flows or men or women who have orinary incontinence. The current selection of adult briefs is, however, unattractive due to their bulk, which makes their concealment under outer clothing difficult or impossible. Further disadvantages of wearing the adult briefs (and sanitary pads) include prolonged exposure to wetness which frequently results in discomfort, irritant dermatitis, and/or infections.

Further, because most pads/tampons and adult briefs are disposable and not reusable, the cost of purchasing the products may be an economic burden since it may add up to a significant sum (e.g., more than S900) per year. Additionally, the waste produced is environmentally unsustainable. Moreover, the pads/tampons may be occasionally positioned incorrectly (e.g., too far up or back) and the adult briefs may be wrapped too loosely, both resulting in severe leakage.

Conventionally, "period underwear" has been designed to reduce the risk of leakage by providing a leak-proof barrier (e.g., a laminated fabric) outside of the undergarment or a thick absorbent pad in the crotch area; such products, however, have limited use because the leak-proof barrier is generally in contact with the skin and may create irritation thereof, and the thick absorbent pad is bulky and uncomfortable to wear. Additionally, the leak-proof barrier and thick absorbent pad may entrap moisture within the undergarment, thereby increasing the risk of yeast infections.

EP0558351 (A1) describes a moisture-management garment having a moisture management panel constructed of a moisture management fabric 10 which extends generally between a crotch area of the garment and a waist area of the garment. The moisture management panel includes a first fabric 11 having an inner moisture transport fabric layer 12 constructed of hydrophobic yarns for residing in skin contact during garment wear and an inner dispersal fabric layer 13 constructed of hydrophilic yarns and positioned adjacent to the inner moisture transport layer to receive and disperse moisture transported to it by the inner moisture transport layer. A second fabric 14 is provided having an outer moister vaporization fabric layer 15 constructed of hydrophobic yarns which is the outermost surface of the moisture management fabric during wear, and an outer moisture dispersal fabric layer 16 constructed of hydrophilic yarns and positioned adjacent to the outer moisture vaporization layer 15. An intermediate wicking insert 20 is positioned between the first and second fabrics 11,14 and extends along the garment from the area of the crotch to the area of the waist to wick moisture from the area of the crotch towards the area of the waist of the garment.

Consequently, there is a need for undergarments that eliminate the need for using disposable pads/tampons and adult briefs while effectively absorbing liquid flow and reducing the risk of leaks. Additionally, it is desirable that the undergarments provide stain prevention, moisture-wicking, antimicrobial management, and skin comfort.

### SUMMARY

The present disclosure provides an undergarment as detailed in claim 1. Advantageous features are provided in dependent claims.

In various embodiments, the present invention includes an undergarment that is leak-proof, moisture wicking, attractive, antimicrobial, and comfortable to wear. The undergarment may also be stain-proof and/or treated with a microbial agent Additionally, the undergarment provides sufficient and effective liquid absorption; the need for using disposable tampons/pads in conjunction with the undergarment is thus eliminated (though their use with embodiments of the present invention is not prohibited). More specifically, the undergarment may include a fabric body constructed of one or more layers and a multilayer pad that is integrated with the interior surface of the fabric body. Because each layer of the multilayer pad is sufficiently thin (e.g., 100-500 g/m²) and the multilayer pad may be fully integrated with the fabric body (e.g., one layer of the pad may form a part of the fabric body), the multilayer pad does not deform the structure of the fabric body (and the overall undergarment) to thereby lessen its visual appeal and/or allow the outline or shape of the pad to be visible through outer clothing). Addtionally, the stitches for integrating the multi layer pad with the fabric body may be hidden inside the fabric body such that the entire pad appears to be seamless and is thereby undetectable from the exterior of the undergarment to the wearer or other viewers. As used herein, undetectable means that the structure/material cannot be detected, or is very difficult to detect, by touch and/or is hidden from sight

Each layer of the multilayer pad may be positioned within the fabric body in a crotch region that is defined by a pair of leg openings. Alternatively, the layer(s) may extend beyond the crotch region (e.g., up to the waist opening of the undergarment). The multilayer pad includes four layers: the innermost layer may be treated with a nano-particle solution (e.g., polyethylene oxide or silicone) to wick moisture and an antimicrobial material (e.g., a silver nitrate solution) is applied thereto for preventing infections; the second layer is made of, for example, cotton or other materials, that can efficiently absorb the moisture or liquid; the third layer includes, for example, cotton bonder with a moisture-impermeable material (e.g., wax or breathable urethane) on the outer surface (i.e., facing away the body) to prevent leakage; and the outermost layer is made of a supportive, elasticized fabric blend that has a thicker texture such that the cuts and stitched seams of the innermost, moisture-wicking layer and middle layers (i.e, the second, moisture-absorbing layer and the third, moisture-impermeable layer) are effectively camouflaged, and are thereby not viewable through the garment. In a preferable embodiment, the multilayers of the pad are stitched together using a densely (or tightly) sewn seam; this can further prevent leakage of the liquid. In one embodiment, the innermost layer is a dark color (e.g., black) such that any stains are nonobvious or invisible.

In some embodiments, the undergarment includes one or more pad pockets that each allows a pad to be inserted therein. The insertable pad can be frequently removed from the pocket as a single unit for sanitary reasons. In one embodiment, the insertable pad is washable and reusable for reducing the wearers' cost and providing an environmentally friendly alternative. In another embodiment, the insertable pad is disposable for offering convenience to the wearers during special occasions, e.g., traveling. Again, the insertable pad may include an antimicrobial coating layer, a moisture-wicking layer, a moisture-absorbent layer, and/or a moisture-impermeable layer to provide the corresponding function of each layer. In some embodiments, the undergarment includes one or more pockets located in the front and/or back of the undergarment for retaining removable heating pads that may provide cramp relief during menstruation.

Accordingly, in one aspect, the invention pertains to an undergarment including a fabric body having a waist opening and a pair of leg openings defining a crotch region therebetween, and a multilayer leak-proof pad stitched to an interior surface of the fabric body and extending over at least the crotch area. The leak-proof pad includes a moisture-impermeable layer in contact with the fabric body, a moisture-absorbent layer thereover, and a moisture-wicking layer over the absorption layer. The multilayer leak-proof pad further comprises an anti-microbial layer over the wicking layer. The multilayer leak-proof pad extends over the crotch region and includes pairs of opposed curving peripheral edges; the first pair of edges extends along the leg openings proximate to the crotch region and the second pair of edges is located beyond the crotch region and flaring outwardly. Additionally, the moisture-absorbent layer, the moisture-wicking layer and the antimicrobial layer may extend as a unitary panel beyond the polymer layer to a waistband around the waist opening. One or more rear segments of the unitary panel have opposed peripheral edges tapering toward the waistband.

In various embodiments, the undergarment further includes an extension region stitched to and extending beyond the waistband; the extension region includes one or more pockets for receiving one or more removable heating pads. In one implementation, the pocket has an entrance along a side edge thereof. Additionally, the moisture-absorbent layer, the moisture-wicking layer and the antimicrobial layer may be removable as a unitary insert, and the undergarment may include a pocket for removably retaining the insert.

In some embodiments, one or both surfaces of the moisture-wicking layer is treated with a hydrophilic composition and/or a hydrophobic composition to create a net hydrophilic gradient over the moisture-wicking layer. The moisture-wicking layer may include at least 51% cotton; the moisture-absorbent layer may include cotton; the moisture-impermeable polymer layer may include breathable urethane or wax; and the fabric body may include an elastic material.

A second non-claimed aspect relates to a method of manufacturing an undergarment. In various embodiments, the method includes providing a multilayer leak-proof pad having a moisture-impermeable polymer layer, a moisture-absorbent layer, and a moisture-wicking layer; and stitching the multilayer leak-proof pad to an interior surface of a fabric body to form the undergarment In one embodiment, the method includes forming the multilayer leak-proof pad to cover a crotch region defined by a pair of leg openings. In another embodiment, the method includes forming the multilayer leak-proof pad to cover a crotch region defined by a pair of leg openings and an extension region extending beyond the crotch region to a waistband. Additionally, the method may include creating one or more pockets in the extension region for receiving one or more removable heating pads.

In one implementation, the method includes creating one or more pockets in the undergarment for retaining the multilayer leak-proof pad. The method may further include treating the moisture-wicking layer with an antimicrobial solution, a hydrophilic composition and/or a hydrophobic composition.

Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

### BRIEF DESCRIPTION OF THE DRAWING

In the drawings, like reference characters generally refer to the same pans throughout the different views. Also, the drawings are not necessarily to scale, with an emphasis instead generally being placed upon illustrating the principles of the invention. In the following description, various embodiments of the present invention are described with reference to the following drawings, in which:
FIGS. 1A, 1B, 1C, and 1D schematically depict undergarments in accordance with various embodiments of the invention;
FIG. 2 depicts a structure of a multilayer leak-proof pad in accordance with an embodiment of the invention;
FIGS. 3A, 3B, 3C, and 3D schematically depict the cuts, shapes, and sizes of the multilayer pad in accordance with various embodiments of the invention;
FIGS. 4A, 4B, and 4C schematically depict the seams of the multilayer pad stitched to the undergarment in accordance with an embodiment of the invention;
FIGS. 5A and 5B depict the positions of an insertable, removeable pad and the pocket a non-claimed that retains the removable pad in accordance with a non-claimed embodiment;
FIGS. 6A and.6B depict an overhead and isometric view of an undergarment in accordance with a non-claimed embodiment; and
FIGS. 7A, 7B, and 7C depict undergarments having various sizes and shapes of multilayer pads in accordance with non-claimed embodiments.

### DETAILED DESCRIPTION

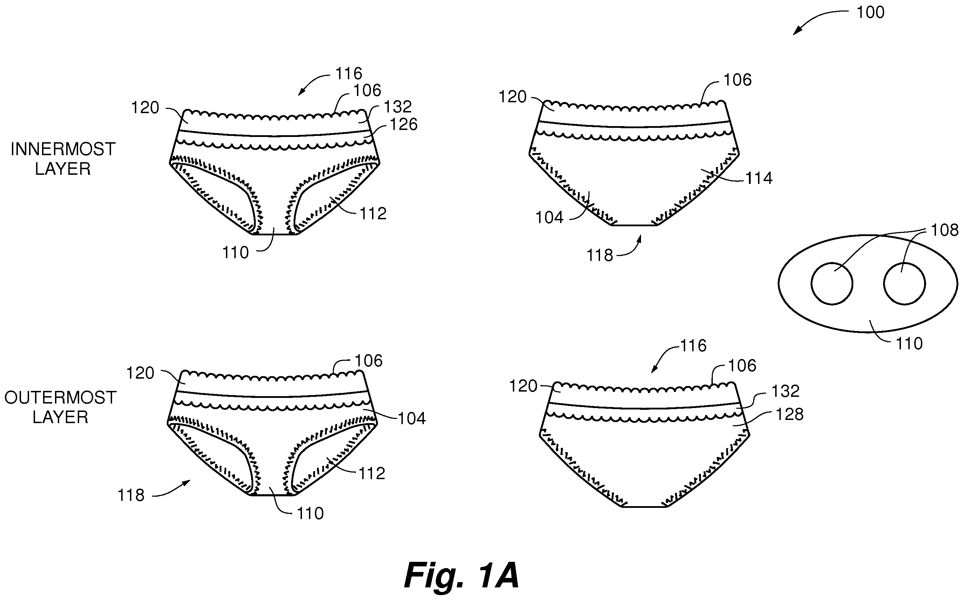

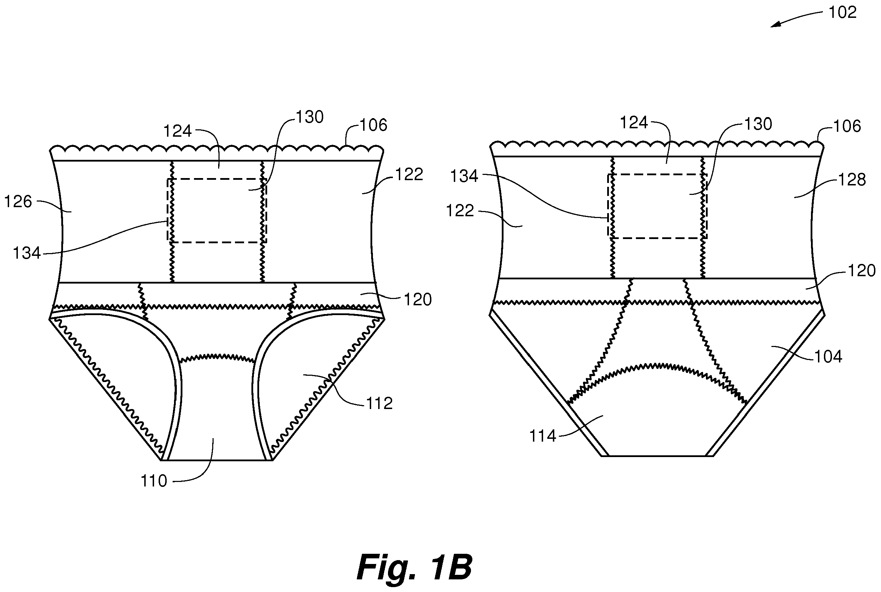

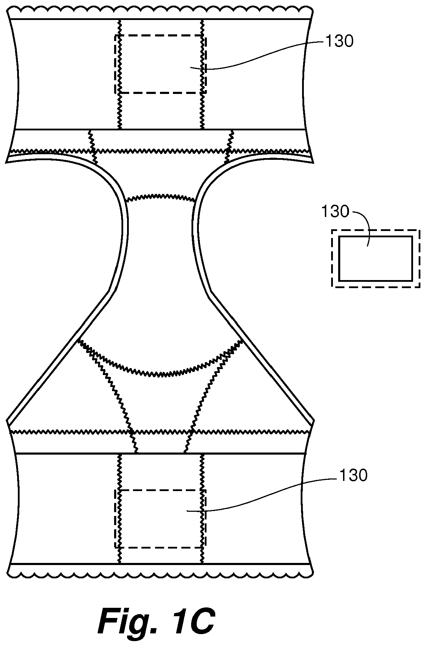

FIGS. 1A and 1B illustrate exemplary undergarments 100, 102 in accordance with various embodiments of the invention; FIG. 1C depicts a view of the material/fabric of the undergarment in a single two-dimensional plane. The undergarments 100, 102 include a fabric body 104 having a waist opening 106 and a pair of leg openings 108 defining a crotch area 110 therebetween that covers some or all of the genital area of a wearer; the undergarments 100, 102 further include an interior surface 112 (i.e., a surface of the fabric body 104 that wholly or partially contacts the body of the wearer when the undergarments 100, 102 are worn) and an exterior surface 114 (i.e., a surface of the fabric body 104 distal the interior surface 112 with respect to the body of the wearer such that some or all of the exterior surface 114 does not contact the body of the wearer). The undergarments 100, 102 further include a multilayer pad, as described in greater detail below, that is attached via stitching to the interior surface 112 of the fabric body 104. The multilayer pad is disposed in the crotch area 110; the pad may extend over some or all of the crotch area 110 and/or extend beyond the crotch area 110. Although FIGS. 1A and 1B display exemplary embodiments of the present invention, any cut, size, style, color, type of under- or outer-garments are within the scope of the present invention. For example, the fabric body 104 may include running shorts, bicycle shorts, swimwear, yoga pants, body-shape-altering "stretch" pants or shorts, sport or "performance" underwear, or any other such type or style of garment.

The fabric body 104 may include one or more layers. In one embodiment, the fabric body 104 includes one layer, and the multilayer pad, as described in greater detail below, is attached to the crotch area 110 of the fabric body 104. In another embodiment, the fabric body includes two layers: an inner fabric-body layer 116 that wholly or partially contacts the body of the wearer and an outer fabric-body layer 118 distal the inner fabric-body layer 106 with respect to the wearer's body. In various embodiments, the outer fabric-body layer 118 exhibits one or more of various colors, patterns, or designs (e.g., black, white, pink, etc.) to provide choices to the wearer; the inner fabric-body layer 116 may exhibit a dark color (e.g., black or dark grey) for providing stain-resistance such that any stain thereon is invisible, unnoticeable, or reduced in visibility or noticeability to the wearer. If the fabric body 104 includes two or more layers, all, some, or none of the inner layer or layers may be visible to the wearer or other observer when the undergarments 100, 102 are worn. Any number of layers of the fabric body 104 is within the scope of the present invention, and any of the layers may be cut to the full size of the outer fabric-body layer 118 or cut to less than the full size thereof.

In one embodiment, the inner fabric-body layer 116 is made of or includes a thin fabric (e.g., cotton or a cotton blend, a synthetic material, or any other material) and the outer fabric-body layer 118 is made of or includes a thick or thicker (with respect to the inner fabric-body layer 116) fabric (e.g., a thicker cotton or cotton blend layer, an elasticized fabric blend such as SPANDEX that may provide support to the wearer and/or alter the shape of the body of the wearer, or any other material). The inner fabric-body layer 116 may be cut to the full shape of the fabric-body 104 or be cut to less than the full shape of the fabric-body 104. For example, the inner fabric-body layer 116 may be cut to extend to cover only the crotch region 110. The inner fabric-body layer 116 may be densely stitched to the inner surface of the outer fabric-body layer 118 to thereby prevent leakage of fluids from the body of the wearer to a point on the outer surface 114 of the undergarments 100, 102 (or other point on the body of the wearer and/or the wearer's clothes) visible to the wearer or other viewer and/or to any point on the inner surface of the undergarments 100, 102 outside of the crotch area 110 that may be felt or otherwise sensed by a wearer.

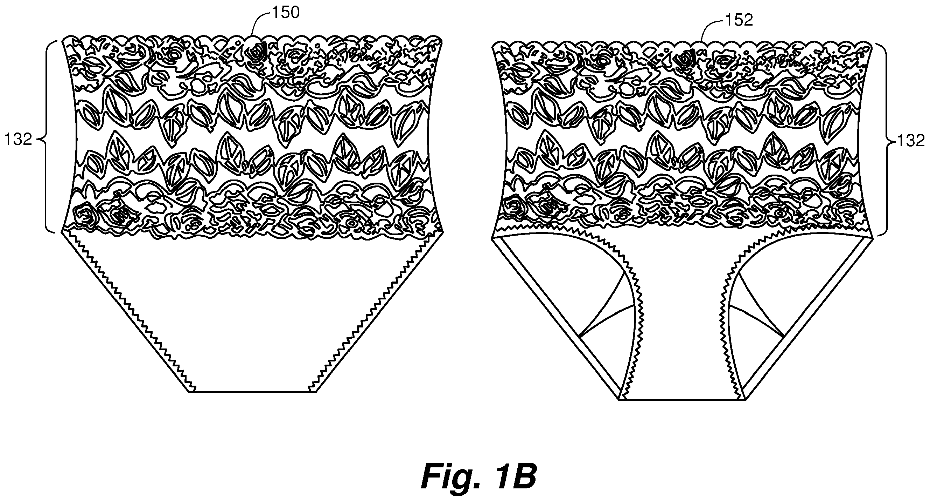

The undergarment may have a low cut, i.e., the waistband 120 may be lower than the wearer's waist (typically, at a location at or near the hips of the wearer) as depicted in FIG. 1A or have a high cut, i.e., including an extension region 122 extending above the waistband 120. Any height of the waistband 120 is within the scope of the present invention. In various embodiments, and in particular with reference to the embodiments of FIGS. 1B, 1C, or other embodiments having waistband 120 above the wearer's waist, one or more interior pockets 124 are disposed in the front panel 126 and/or back panel 128 of the inner layer 116 for receiving and holding in position one or more removable pad 130. The interior pocket 124 is preferably located beneath the waist opening 106 and, in some embodiments, beneath a layer of decorative lace 132 (or other adornment; rear 150 and front 152 views of one example of such a decorative layer 132 appear in FIG. 1D) and thereby may be particularly suitable for implementations in which the undergarment has a high cut, such as the embodiment depicted in FIG. 1B. The removable pad 130 may be or include one or more heating pads that provide warming heat to the wearer for reducing cramps during menstruation. The heating pads may provide heat via a chemical reaction, via prior heating and later retention of said heat, or any other means. In some embodiments, the interior pocket124 has an entrance or opening along a side edge 134 (i.e., parallel or substantially parallel to a vertical or longer axis of the interior pocket 124) for receiving the removable pad 130 such that the removable pad 130 is securely retained therewithin. For example, the removable pad 130 remains secured inside the interior pocket 124 when the wearer moves, accelerates or decelerates, or otherwise changes positions (e.g., from standing up to crouching down) and/or puts on or takes off the undergarment. Any orientation of the opening (e.g., perpendicular or diagonal with respect to the vertical or longer axis of the interior pocket 124) is within the scope of the current invention. The interior pocket 124 may further include a material or mechanism (e.g., hook-and-loop fasteners) to secure the removable pad 130 with the pocket 124 and/or a material or mechanism to secure the opening of the pocket 124 closed to thereby secure the removable pad 130 inside.

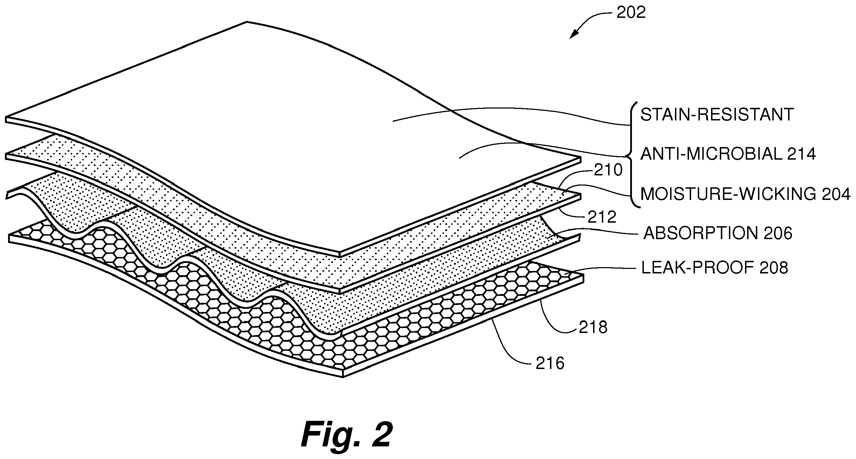

The undergarment includes a multilayer pad to absorb a liquid flow (e.g., bodily secretions, discharges, urine, or menstrual fluids) and thereby prevent or reduce leakage thereof. Referring to FIG. 2, the multilayer pad 202 includes three pad layers 204, 206, 208 (e.g., 100-300 g/m²); each pad layer 204,206,208 may extend over at least some or all of the crotch region 110. The pad layers include a moisture-wicking pad layer 204 disposed closest to the body of the wearer, a moisture-absorbent pad layer 206 disposed next closest to the body of the wearer (i.e., distal the moisture-wicking pad layer 204 with respect to the body of the wearer), and a moisture-impermeable pad layer 208 disposed distal the moisture-absorbent pad layer 206 (i.e., furthest from the body of the wearer). The composition, properties, and functions of each of the pad layers 204, 206, 208 will now be described.

The moisture-wicking pad layer 204 may be made of or include a moisture-wicking material that transfers (and/or facilitates the transfer) of moisture or liquid from its inner surface 210 (i.e., the surface facing the wearer) to its outer surface 212 (i.e., the surface facing away the wearer and facing and/or in contact with the moisture-absorbent pad layer 206). In one embodiment, the inner fabric-body layer 116 of the undergarment 100, as described above, provides the moisture-wicking pad layer 204. That is, the moisture-wicking pad layer 204 is part of the inner fabric-body layer 116, which itself exhibits moisture-wicking properties throughout the fabric body 104 and/or just in the crotch area 110. In another embodiment, the moisture-wicking pad layer 204 is a layer separate and distinct from the inner fabric-body layer 116 and may be attached thereto using, for example, stitches. The inner fabric-body layer 116 may be moisture-wicking even if the moisture-wicking pad layer 204 is a separate layer.

The moisture-wicking pad layer 204 may comprise, consist of, or include any moisture-wicking material known in the art. The material may be naturally moisture-wicking and/or be treated to become moisture-wicking. In one embodiment, moisture-wicking pad layer 204 includes at least 51% cotton. One or both inner and outer surfaces 210,212 of the moisture-wicking pad layer 204 may be treated with a hydrophilic composition or material (e.g., polyethylene oxide, polyvinyl alcohol, polyacrylamide, poly acrylic acid, polyvinyl pyrrolidone, hydrophilic silicones, or hydrophilic polyurethanes) and/or a hydrophobic composition or material (e.g., silicones, polyfluoroalkylacrylates, polyacrylates, polyurethanes, or waxes) to create a net hydrophilic gradient over the moisture-wicking pad layer 204 (i.e., the inner surface 210 may be less hydrophilic (i.e.. more hydrophobic) whereas the outer surface 212 may be more hydrophilic). For example, the inner surface 210 may be treated with a hydrophobic material and/or the outer surface 212 may be treated with a hydrophilic material. As a result, a combination of a rushing" force generated by any hydrophobic properties of the inner surface 210 (and/or portions of the moisture-wicking pad layer 204 near the inner surface 210) and a "pulling" force generated by any hydrophilic properties of the outer surface 212 (and/or portions of the moisture-wicking pad layer 204 near the outer surface 212) may wick any moisture or liquid through the moisture-wicking pad layer 204 and away from the wearer (i.e., from the inner surface 210 to the outer surface 212). The hydrophilic and hydrophobic compositions may be applied to the moisture-wicking pad layer 204 using any conventional methods; see, e.g., U.S. Patent No. 7,842,625. In one embodiment, the rate of wicking through the moisture-wicking pad layer 204 may be controlled to be faster or slower. The rate may be set at a maximum rate of absorption of the moisture-absorbent pad layer 206 to thereby ensure that all, or a significant percentage of, the moisture wicking through the moisture-wicking pad layer 204 is absorbed by the moisture-absorbent pad layer 206 and does not, for example, leak beyond the confines of the undergarment. The rate of wicking may be controlled by the density, thickness, or composition of the moisture-wicking pad layer 204 and/or by the amount and type of hydrophobic and/or hydrophilic material applied to said layer. In another embodiment, the rate of wicking may be set such that the inner surface of the moisture-wicking pad layer 204 feels "dry" or mostly dry to the wearer while the outer surface of the moisture-wicking pad layer 204 feels wet. Additionally, the moisture-wicking pad layer 204 includes an antimicrobial layer 214 by applying an antimicrobial solution, such as a silver nitrate solution, to the inner surface 210 thereof to provide an antimicrobial barrier, thereby reducing infections caused by microorganisms. The antimicrobial layer 214 may be disposed at the inner surface 210 of the moisture-wicking pad layer 204 and/or be distributed throughout the moisture-wicking pad layer 204. The hydrophilic composition, hydrophobic composition, and/or antimicrobial solution may be applied to the entire innermost layer 204 or a part thereof (such as the portion that has an absorption layer thereover as further described below).

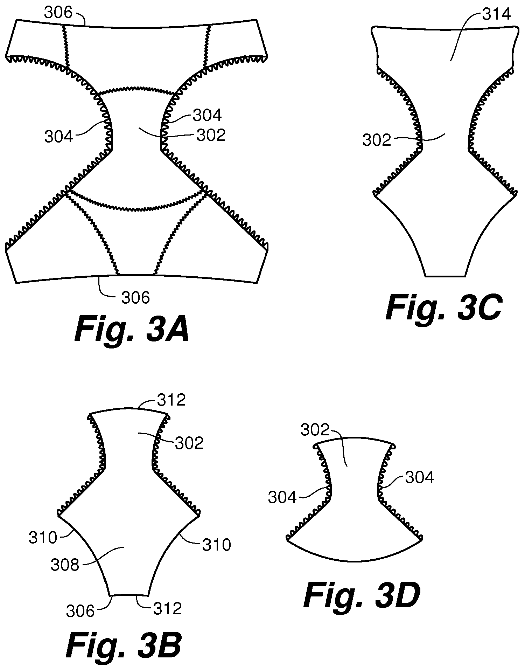

The moisture-absorbent pad layer 206 may comprise, consist of, or include any liquid-absorbing material known in the art (e.g., cotton, a cotton blend, foam, a synthetic material, absorbent polymeric foam, a nanotechnology-based or -produced material, or any other moisture-absorbent material) and may have a weight of 180-300 g/m². Referring to FIGS. 3A-3D, the moisture-absorbent pad layer 206 may have various shapes and/or sizes; a small size (FIG. 3D), a medium size (FIG. 3B), or a large size (FIG. 3C). Referring to FIG. 3A, for example, the moisture-absorbent pad layer 206 may have a curved-shape and be disposed along the crotch area 302 between the leg holes 304. Alternatively, referring to FIG. 3B, the moisture-absorbent pad layer 206 may extend beyond the crotch region 302 and up the back panel (such as, to the waistband 306 around the waist opening) to provide extra absorption capabilities for wearers when they sleep or recline. For example, the extension region 308 may include two pairs of opposing curved peripheral edges 310, 312; the first pair 310 of the edges may extend along the leg openings 304 proximate the crotch region 302 and the second pair 312 of the edges may be located beyond the crotch region 302 and flare outward. In one embodiment, the rear segment of the moisture-absorbent pad layer 206 has opposing peripheral edges that are tapered toward the waistband 306; this design offers effective fluid absorption to the most important areas while economically saving the fabric material and reducing any bulky feeling for the wearers. Referring to FIG. 3C, in another embodiment, the moisture-absorbent pad layer 206 may extend up the front panel 314 to provide additional regions for liquid absorption; this provides the wearer the most absorption capability and helps prevent liquid leakage when, for example, the wearer leans forward. Though the preceding discussion was made with reference to the moisture-absorbent pad layer 206, the other layers (e.g., moisture-wicking pad layer 204 and the moisture-impermeable pad layer 208) may have the same or similar sizes and shapes.

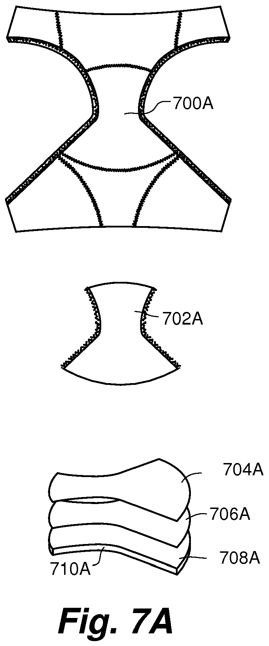

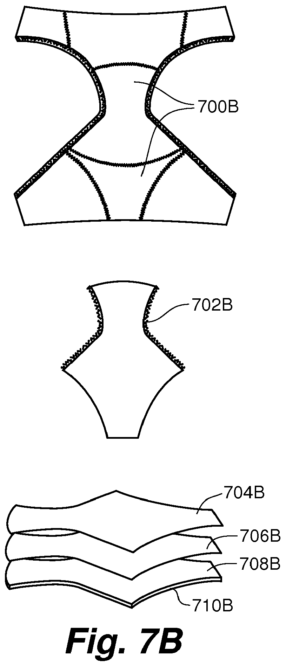

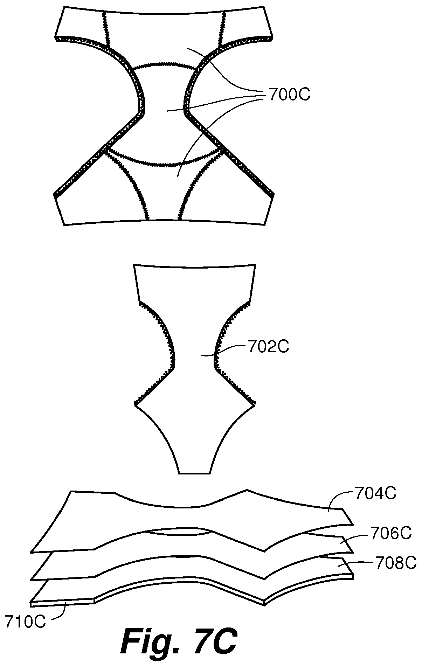

The moisture-impermeable pad layer 208 may comprise, consist of, or include any wholly or partially moisture-blocking material known in the art. Referring again to FIG. 2, in some embodiments, the moisture-impermeable pad layer 208 includes a moisture-impermeable polymer layer 216 (e.g., breathable urethane or wax) on the surface 218 facing away from the wearer (and/or in contact with the outer fabric-body layer 104 of the undergarment) to create a leak-proof barrier against leakage of the liquid flow. The moisture-impermeable pad layer 208 may further include a layer, material, or coating (such as a soft fabric layer) between the moisture-impermeable polymer layer 216 and the wearer. Because the moisture-impermeable polymer layer 216 is positioned on the outer surface 218 of the soft fabric (with respect to the wearer), any discomfort due to the polymer, plastic, wax cotton, or other such moisture-impermeable material pressing against the wearer's skin is thus minimized or eliminated. Additionally, the moisture-impermeable pad layer 208 may be shaped based on the shape of the moisture-absorbent pad layer 206. The moisture-impermeable pad layer 208 is disposed only in the crotch area 110 (in accordance with the variations shown in, for example, FIGS. 7A, 7B, 7C, as described in greater detail below), to allow other areas of the fabric body of the undergarment not covered by the moisture-impermeable pad layer 208 to "breathe" (i.e. to allow moisture to pass freely), to thereby enhance the comfort of the wearer. The moisture-impermeable pad layer 208 and the moisture-absorbent pad layer 206 may have identical or similar shapes but different sizes. For example, the moisture-impermeable pad layer 208 may be larger than the moisture-absorbent pad layer 206 to avoid leakage from any point on the border of the moisture-absorbent pad layer 206 or may be smaller than the moisture-absorbent pad layer 206 to provide leakage protection only in the areas that are most likely to leak.

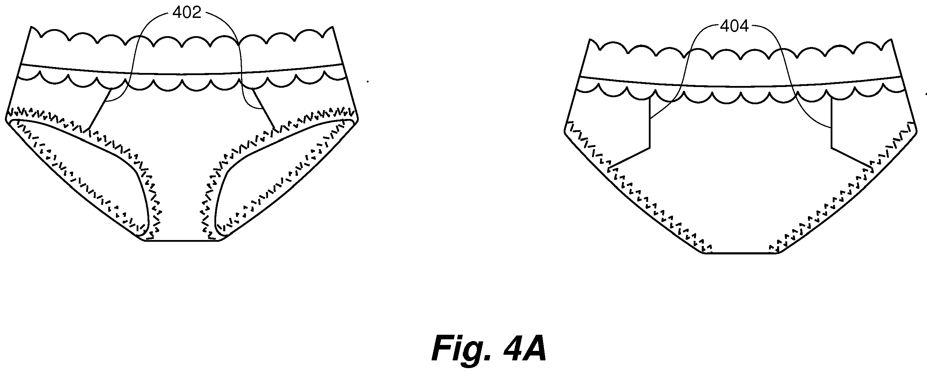

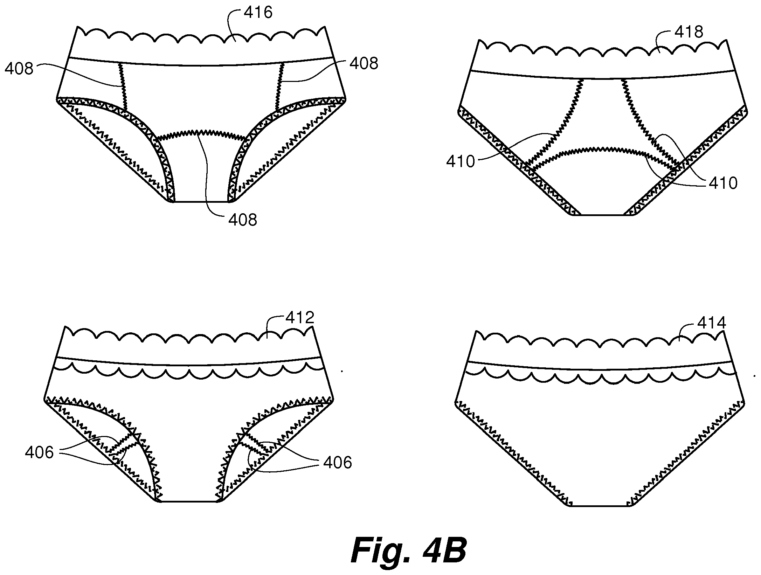

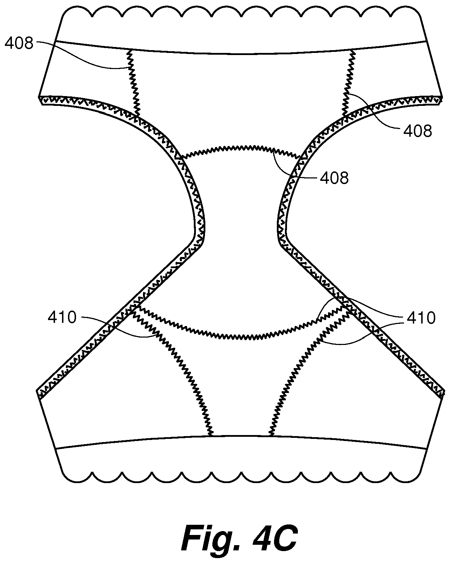

In various embodiments, the outer fabric-body layer 118 of the garment forms the outermost layer of the multilayer pad. Because the outer fabric-body layer 118 may be made of thick fabric (e.g., 100-500 g/m²), it is capable of covering the patterns of the middle pad layers (i.e., the moisture-impermeable pad layer 208 and the moisture-absorbent pad layer 206) and the moisture-wicking pad layer 204 such that the shapes of the inner pad layers 204, 206, 208 are not visible through the undergarment. If the inner fabric-body layer 116 of the garment forms the innermost pad layer (i.e., the moisture-wicking layer 204) of the pad 202, the middle pad layers 206,208 are stitched to the surface of the innermost pad layer 204 and/or the outermost fabric-body layer 118 prior to sewing the inner and outer fabric-body layers 116, 118 of the undergarment together such that the stitch marks of the multilayer pads 202 are visible internally but not externally. Similarly, when the inner fabric-body layer 116 of the garment and the innermost layer of the pad are two separate layers, the moisture-wicking pad layer 204 and middle pad layers 206, 208 may be stitched to the inner fabric-body layer 116 and/or the outer fabric-body layer 118 of the undergarment prior to finishing the undergarment by sewing the inner and outer fabric-body layers 116,118 together. Referring to FIGS. 4A, 4B, and 4C, lines 402,404,406,408,410 indicate various exemplary methods of stitching the middle pad layers 206, 208 (and moisture-wicking pad layer 204) to the inner fabric-body layer 116 and/or outer fabric-body layer 118 of the undergarment to ensure effective leak protection as well as to ensure breathability of the entire garment In one embodiment, the stitching lines 406, 408, 410 are not visible from exterior front 412 and rear 414 views of the undergarment; the stitching may be visible from "inside-out" front 416 and rear 418 views of the undergarment. In another embodiment, the stitching lines 406, 408, 410 are not visible in the inside-out views 416, 418. Stitching along the lines 406, 408, and/or 410 illustrated in FIG. 4B may, in particular provided enhanced comfort to a wearer, provide additional support for the multi-layer pad, and/or provide additional leak resistance. In a preferable embodiment, the seams that hold together the multiple layers of the pad and/or the inner and outer fabric-body layers of the undergarment are densely or tightly stitched such that the available surface area for leakage is reduced. Because the overall thickness of the multilayer pad is sufficiently thin (e.g., up to one centimeter or up to several centimeters), the multilayer pad does not change the shape of the undergarment and may not even be noticeable to the wearer or other viewer. Additionally, the multilayer pad and undergarment may be made of conventional knitted fabric materials, such as a blend of NYLON, LYCRA, SPANDEX, silk, cotton, etc., and therefore may be manufactured using conventional stitching approaches for fabrics.

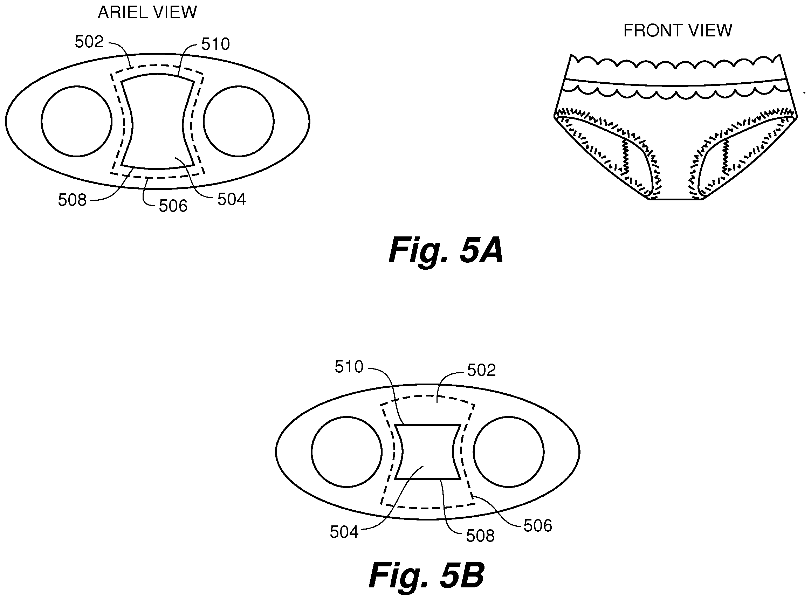

Referring to FIGS. 5A and 5B, the inner fabric-body layer 116 of the garment includes a thin fabric, an area 510 of which is cut open to form one or more pocket spaces 502 enclosed by the outer fabric-body layer 118. One or more removable pads 504 having a moisture-wicking pad layer 204 (and, optionally, having the antimicrobial application thereon), a moisture-absorbent pad layer 206 and a moisture-impermeable pad layer 208 act as unitary inserts that can seamlessly fit within the pocket space 502. The pocket(s) 502 that retains the removable pad(s) 504 may be located in the crotch region only or may extend therebeyond. Dashed lines 506 indicate the seams of the pocket(s) 502: the removable pad 504 may extend beneath the inner fabric-body layer 116 until its borders lie at or near the seams 506 of the pocket 502. The cut-out areas 510 may be used to insert the removable pad 504 within the pocket 502. FIG. 5A depicts a larger cut-out area 510, which may make insertion/removal of the pad 504 easier for a wearer, while the FIG. 5B depicts a smaller cut-out area 510, which may better retain the pad 504 against undesirable movement. In an alternative arrangement, the moisture-impermeable pad layer 208 is integrated with the fabric body, i.e., the outer fabric-body layer 118, and is not removable; the insertable pad 504 then includes the moisture-wicking pad layer 204 (and optionally the antimicrobial application thereon) and the moisture-absorbent pad layer 206. The insertable pad 504 may have a size similar to or smaller than the moisture-impermeable pad layer 208; alternatively, the insertable pad 504 may extend as a unitary panel beyond the moisture-impermeable pad layer 208. If the inner fabric-body layer 116 forms the moisture-wicking pad layer 204, a pocket space may be created between the moisture-wicking pad layer 204 and the outer fabric-body layer 118. In this case, the moisture-absorbent pad layer 206 may be coated with the antimicrobial solution, and again, the moisture-absorbent pad layer 206 and the moisture-impermeable pad layer 208 may form the removable pad 504 that can be inserted into the pocket space 502. Because the insertable pads 504 may be removed and changed frequently, they may be preferable to the wearer for sanitary reasons. Addtionally, since the insertable pads 504 are washable and reuseable, they provide a solution for reducing the wearer's cost and are also environmentally-friendly.

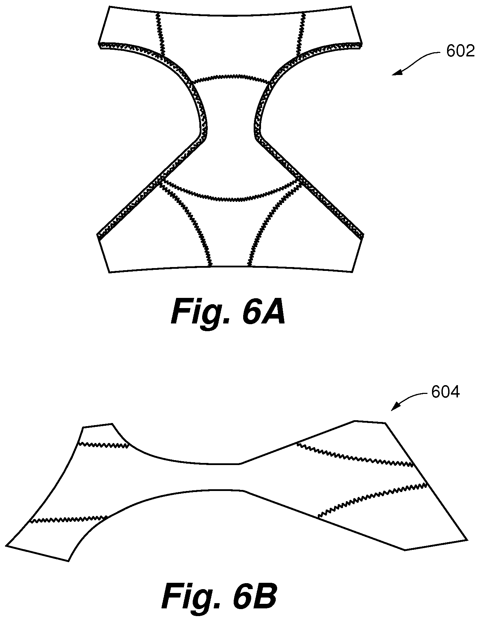

FIGS. 6A and 6B depict overhead and isometric views of undergarments 602, 604. FIGS. 7A, 7B, and 7C depict undergarments 700A. 700B, 700C and associated multilayer pads 702A, 702B, and 702C having various sizes and shapes. The first pad 702A may be used for light-flow days, the second pad 702B may be used for medium-flow days, and the third pad 702C may be used for heavy-flow days. Each multilayer pad 702A, 702B, 702C may have three layers 704A, 706A, 708A; 704B, 706B, 708B; 704C, 706C, 708C, respectively. The first layer 704A, 704B, 704C may be the moisture-wicking pad layer, as described above (and may also include an anti-microbial layer or treatment, as also described above). The second layer 706A, 706B, 706C may be the moisture-absorbent layer, and the third layer 708A, 708B, 708C may be the moisture-impermeable layer, as described above. The third layer 708A, 708B, 708C may include a ridge 710A, 710B, 710C running around all or a portion of the periphery of the third layer 708A, 708B, 708C; this ridge may be made of the same material as the moisture-impermeable pad layer (e.g., polyurethane or other moisture-impermeable material) or any other material. The height of the ridge 710A, 710B, 710C with respect to the plane of the third layer 708A, 708B, 708C may be greater than or equal to the thickness of the second layer 706A, 706B, 706C and/or the first layer 704A, 704B, 704C to thereby prevent or deter moisture leaks from the second layer 706A, 706B, 706C.

The undergarment of the current invention includes various advantages (e.g., leak prevention, stain prevention, moisture-wicking comfort, antimicrobial management, cramp relief and liquid absorption), thereby providing the wearer with a supportive, comfortable and effective way to manage her needs; whereas the conventionally available products either have poor liquid absorption or are too thick or bulky to wear comfortably. In addition, the stitches for integrating the multilayer pad with the fabric body in the undergarment of the current invention may be hidden inside the fabric body such that the entire pad appears to be seamless and is thereby invisible to the wearer. Further, the various shapes of the multilayer pad provide various options to balance the need of sufficient liquid absorption and comfort (e.g., breathable and less bulky) of the undergarment.

The undergarment of the current invention need not be comprised only of the four layers as described above; other layers may be additionally or alternatively incorporated in between or outside the four-layer structure for providing additional absorption support, decoration, or any other functionality. In addition, the ordering of the layers in the above-described four-layer structure may be interchanged to the extent that the key functionalities, including moisture wicking, leak and stain resistant, and antimicrobial, are retained and further supported by such an ordering.

Additionally, in various alternative embodiments, the fibrous materials described in specific embodiments herein may be substituted by materials that would be recognizable as compatible by those skilled in the art. Further, the chemicals compounds, stains, dyes and other treatments or elements of the garment, its design, or the process by which it is created may be substituted with other known materials recognized by those skilled in the art, including but not limited to hydrophilic elements, hydrophobic elements, sealants and others. The various components or subparts of an embodiment or the embodiment itself may be substituted and used with other subparts of other embodiments and various combinations thereof provided the claimed functionalities or general purpose of the design is preserved.

Certain embodiments of the present invention were described above. It is, however, expressly noted that the present invention is not limited to those embodiments, but rather the intention is that additions and modifications to what was expressly described herein are also included within the scope of the invention. Moreover, it is to be understood that the features of the various embodiments described herein were not mutually exclusive and can exist in various combinations and permutations, even if such combinations or permutations were not made express herein, without departing from the scope of the invention. In fact, variations, modifications, and other implementations of what was described herein will occur to those of ordinary skill in the art without departing from the scope of the invention. As such, the invention is not to be defined only by the preceding illustrative description.

## Claims

1. An undergarment (100; 102), comprising:
a fabric body (104) having a waist opening (106) and a pair of leg openings (108; 304) defining a crotch region (110; 302) therebetween; and
a multilayer leak-proof pad stitched to an interior surface of the fabric body (104) and extending over at least the crotch region (110), the multilayer leak-proof pad comprising a moisture-impermeable layer (208) in contact with the fabric body (104); a moisture-absorbent layer (206) thereover; a moisture-wicking layer (204) over the moisture-absorbent layer (206); and an anti-microbial layer over the moisture-wicking layer (204);
wherein the multilayer leak-proof pad extends over the crotch region and includes pairs of opposed curving peripheral edges, a first pair of edges extending along the leg openings proximate to the crotch region and a second pair of edges being located beyond the crotch region and flaring outwardly;
wherein the moisture-impermeable layer (208) is disposed only in the crotch region, and
wherein the moisture-absorbent layer (206), the moisture-wicking layer (204), and the anti-microbial layer extend as a unitary panel beyond the moisture-impermeable layer (208) up a back panel of the undergarment to a waistband (306) around the waist opening (106), at least a rear segment of the unitary panel having opposed peripheral edges (310) tapering toward the waistband (306).

2. The undergarment (100; 102) of claim 1, further comprising an extension region (122) stitched to and extending above the waistband (306), the extension region (122) comprising at least one pocket (124) for receiving a removable heating pad.

3. The undergarment (100; 102) of claim 2, wherein the pocket (124) has an entrance along a side edge (134) thereof.

4. The undergarment (100; 102) of claim 1, wherein the moisture-wicking layer (204) comprises at least 51% cotton.

5. The undergarment (100; 102) of claim 1, wherein the moisture-absorbent layer (206) comprises cotton or foam.

6. The undergarment (100; 102) of claim 1, wherein the fabric body (104) comprises an elastic material.

7. The undergarment (100; 102) of claim 1, wherein the moisture-impermeable layer (208) comprises a moisture-impermeable polymer layer (216).

8. The undergarment (100; 102) of claim 7, wherein the moisture-impermeable polymer layer (216) comprises breathable urethane or wax.

9. The undergarment (100; 102) of claim 1, wherein the moisture-wicking layer (204) comprises a net hydrophilic gradient, and wherein at least one surface of the moisture-wicking layer (204) is treated with a hydrophilic composition or a hydrophobic composition to create the net hydrophilic gradient.

10. The undergarment (100; 102) of claim 9, wherein the at least one surface of the moisture-wicking layer (204) is treated to be moisture wicking by application of the hydrophilic composition.

11. The undergarment (100; 102) of claim 10, wherein the hydrophilic composition is selected from the group consisting of polyethylene oxide, polyvinyl alcohol, polyacrylamide, poly acrylic acid, polyvinyl pyrrolidone, hydrophilic silicones, and hydrophilic polyurethanes.

12. The undergarment (100; 102) of claim 9, wherein the at least one surface of the moisture-wicking layer (204) is treated to be moisture wicking by application of the hydrophobic composition.

13. The undergarment (100; 102) of claim 12, wherein the hydrophobic composition is selected from the group consisting of silicones, polyfluoroalklacrylates, polyacrylates, polyurethanes, and waxes.

## Patentansprüche

1. Unterbekleidungsstück (100; 102), das Folgendes umfasst:
einen Gewebekörper (104) mit einer Taillenöffnung (106) und einem Paar von Beinöffnungen (108; 304), die einen Schrittbereich (110; 302) dazwischen definieren; und
ein mehrschichtiges, auslaufsicheres Polster, das auf eine innere Oberfläche des Gewebekörpers (104) genäht ist und sich mindestens über den Schrittbereich (110) erstreckt, wobei das mehrschichtige auslaufsichere Polster eine feuchtigkeitsundurchlässige Schicht (208) umfasst, die mit dem Gewebekörper (104) in Kontakt ist; eine feuchtigkeitsabsorbierende Schicht (206) darüber; eine feuchtigkeitstransportierende Schicht (204) über der feuchtigkeitsabsorbierenden Schicht (206) und eine antimikrobielle Schicht über der feuchtigkeitstransportierenden Schicht (204);
wobei das mehrschichtige auslaufsichere Polster sich über den Schrittbereich erstreckt und Paare von gegenüberliegenden gekrümmten Umfangskanten aufweist, wobei sich ein erstes Paar von Kanten entlang der Beinöffnungen in der Nähe des Schrittbereichs erstreckt und ein zweites Paar von Kanten über dem Schrittbereich angeordnet ist und sich nach außen aufweitet;
wobei die feuchtigkeitsundurchlässige Schicht (208) nur im Schrittbereich angeordnet ist und wobei sich die feuchtigkeitsabsorbierende Schicht (206), die feuchtigkeitstransportierende Schicht (204) und die antimikrobielle Schicht als einheitliche Stoffbahn über die feuchtigkeitsundurchlässige Schicht (208) hinaus aufwärts zu einer hinteren Stoffbahn des Unterbekleidungsstücks zu einem Bund (306) um die Taillenöffnung (106) herum erstrecken, wobei mindestens ein hinteres Segment der einheitlichen Stoffbahn gegenüberliegende Umfangskanten (310) aufweist, die sich zu dem Bund (306) hin verjüngen.

2. Unterbekleidungsstück (100; 102) nach Anspruch 1, das ferner einen Erweiterungsbereich (122) umfasst, der an den Bund (306) genäht ist und sich über diesen erstreckt, wobei der Erweiterungsbereich (122) mindestens eine Tasche (124) zur Aufnahme eines entfernbaren Heizkissens umfasst.

3. Unterbekleidungsstück (100; 102) nach Anspruch 2, wobei die Tasche (124) einen Eingang entlang einer Seitenkante (134) derselben aufweist.

4. Unterbekleidungsstück (100; 102) nach Anspruch 1, wobei die feuchtigkeitstransportierende Schicht (204) mindestens 51 % Baumwolle umfasst.

5. Unterbekleidungsstück (100; 102) nach Anspruch 1, wobei die feuchtigkeitsabsorbierende Schicht (206) Baumwolle oder Schaumstoff umfasst.

6. Unterbekleidungsstück (100; 102) nach Anspruch 1, wobei der Gewebekörper (104) ein elastisches Material umfasst.

7. Unterbekleidungsstück (100; 102) nach Anspruch 1, wobei die feuchtigkeitsundurchlässige Schicht (208) eine feuchtigkeitsundurchlässige Polymerschicht (216) umfasst.

8. Unterbekleidungsstück (100; 102) nach Anspruch 7, wobei die feuchtigkeitsundurchlässige Polymerschicht (216) atmungsaktives Urethan oder Wachs umfasst.

9. Unterbekleidungsstück (100; 102) nach Anspruch 1, wobei die feuchtigkeitstransportierende Schicht (204) einen hydrophilen Nettogradienten umfasst und wobei mindestens eine Oberfläche der feuchtigkeitstransportierenden Schicht (204) mit einer hydrophilen Zusammensetzung oder einer hydrophoben Zusammensetzung behandelt ist, um den hydrophilen Nettogradienten zu erzeugen.

10. Unterbekleidungsstück (100; 102) nach Anspruch 9, wobei die mindestens eine Oberfläche der feuchtigkeitstransportierenden Schicht (204) durch Anwendung der hydrophilen Zusammensetzung so behandelt ist, dass sie feuchtigkeitstransportierend ist.

11. Unterbekleidungsstück (100; 102) nach Anspruch 10, wobei die hydrophile Zusammensetzung aus einer Gruppe ausgewählt wird, die aus Folgendem besteht:
Polyethylenoxid, Polyvinylalkohol, Polyacrylamid, Polyacrylsäure, Polyvinylpyrrolidon, hydrophile Silikone und hydrophile Polyurethane.

12. Unterbekleidungsstück (100; 102) nach Anspruch 9, wobei die mindestens eine Oberfläche der feuchtigkeitstransportierenden Schicht (204) durch Anwendung der hydrophoben Zusammensetzung so behandelt ist, dass sie feuchtigkeitstransportierend ist.

13. Unterbekleidungsstück (100; 102) nach Anspruch 12, wobei die hydrophobe Zusammensetzung aus der Gruppe ausgewählt wird, die aus Folgendem besteht: Silicone, Polyfluoralklacrylate, Polyacrylate, Polyurethane und Wachse.

## Revendications

1. Sous-vêtement (100 ; 102), comportant :
un corps de tissu (104) ayant une ouverture au niveau de la taille (106) et une paire d'ouvertures au niveau des jambes (108 ; 304) définissant une région au niveau de l'entrejambe (110 ; 302) entre elles ; et
une serviette antifuite multicouche cousue sur une surface intérieure du corps de tissu (104) et s'étendant sur au moins la région au niveau de l'entrejambe (110), la serviette antifuite multicouche comportant une couche imperméable à l'humidité (208) en contact avec le corps de tissu (104) ; une couche absorbant l'humidité (206) sur celle-ci ; une couche évacuant l'humidité (204) sur la couche absorbant l'humidité (206) ; et une couche antimicrobienne sur la couche évacuant l'humidité (204) ;
dans lequel la serviette antifuite multicouche s'étend sur la région au niveau de l'entrejambe et comprend des paires de bords périphériques courbes opposés, une première paire de bords s'étendant le long des ouvertures pour les jambes à proximité de la région au niveau de l'entrejambe et une deuxième paire de bords se trouvant au-delà de la région au niveau de l'entrejambe et allant en s'évasant vers l'extérieur ;
dans lequel la couche imperméable à l'humidité (208) est disposée uniquement dans la région au niveau de l'entrejambe, et
dans lequel la couche absorbant l'humidité (206), la couche évacuant l'humidité (204), et la couche antimicrobienne s'étendent sous la forme d'un panneau unitaire au-delà de la couche imperméable à l'humidité (208) le long d'un panneau arrière du sous-vêtement jusqu'à une ceinture (306) autour de l'ouverture au niveau de la taille (106), au moins un segment arrière du panneau unitaire ayant des bords périphériques opposés (310) allant en s'effilant vers la ceinture (306).

2. Sous-vêtement (100 ; 102) selon la revendication 1, comportant par ailleurs une région d'extension (122) cousue sur la ceinture (306) et s'étendant au-dessus de celle-ci, la région d'extension (122) comportant au moins une poche (124) servant à recevoir un coussinet chauffant amovible.

3. Sous-vêtement (100 ; 102) selon la revendication 2, dans lequel la poche (124) a une entrée le long d'un bord latéral (134) de celle-ci.

4. Sous-vêtement (100 ; 102) selon la revendication 1, dans lequel la couche évacuant l'humidité (204) comporte au moins 51 % de coton.

5. Sous-vêtement (100 ; 102) selon la revendication 1, dans lequel la couche absorbant l'humidité (206) comporte du coton ou de la mousse.

6. Sous-vêtement (100 ; 102) selon la revendication 1, dans lequel le corps de tissu (104) comporte un matériau élastique.

7. Sous-vêtement (100 ; 102) selon la revendication 1, dans lequel la couche imperméable à l'humidité (208) comporte une couche polymère imperméable à l'humidité (216).

8. Sous-vêtement (100 ; 102) selon la revendication 7, dans lequel la couche polymère imperméable à l'humidité (216) comporte de l'uréthane respirant ou de la cire respirante.

9. Sous-vêtement (100 ; 102) selon la revendication 1, dans lequel la couche évacuant l'humidité (204) comporte un gradient hydrophile net, et dans lequel au moins une surface de la couche évacuant l'humidité (204) est traitée au moyen d'une composition hydrophile ou d'une composition hydrophobe pour créer le gradient hydrophile net.

10. Sous-vêtement (100 ; 102) selon la revendication 9, dans lequel ladite au moins une surface de la couche évacuant l'humidité (204) est traitée à des fins d'évacuation de l'humidité par l'application de la composition hydrophile.

11. Sous-vêtement (100 ; 102) selon la revendication 10, dans lequel la composition hydrophile est sélectionnée dans le groupe constitué par du polyéthylène oxyde, de l'alcool polyvinylique, du polyacrylamide, de l'acide polyacrylique, de la polyvinylpyrrolidone, des silicones hydrophiles, et des polyuréthanes hydrophiles.

12. Sous-vêtement (100 ; 102) selon la revendication 9, dans lequel ladite au moins une surface de la couche évacuant l'humidité (204) est traitée à des fins d'évacuation de l'humidité par l'application de la composition hydrophobe.

13. Sous-vêtement (100 ; 102) selon la revendication 12, dans lequel la composition hydrophobe est sélectionnée dans le groupe constitué par des silicones, des acrylates d'alkyle polyfluorés, des polyacrylates, des polyuréthanes, et des cires.
